# EUROPÄISCHE PATENTSCHRIFT

(11) **EP 3 282 244 B1**
(45) Veröffentlichungstag und Bekanntmachung des Hinweises auf die Patenterteilung: **26.07.2023**
(21) Anmeldenummer: 17183201.7
(22) Anmeldetag: 26.07.2017
(51) Int. Cl.: G01N 21/78, G01N 33/00, G01N 21/64, G01N 21/77

(54) **VORRICHTUNG ZUR ERMITTLUNG DER KONZENTRATION EINES ANALYTEN IN EINEM GASFÖRMIGEN MEDIUM**
DEVICE FOR DETERMINING THE CONCENTRATION OF AN ANALYTE IN A GASEOUS MEDIUM
DISPOSITIF DE DÉTERMINATION DE LA CONCENTRATION D'UN ANALYTE DANS UN MILIEU GAZEUX

(30) Priorität: 11.08.2016 DE 102016114918
(43) Veröffentlichungstag der Anmeldung: 14.02.2018
(73) Patentinhaber: Pyro Science GmbH, 52064 Aachen (DE)
(72) Erfinder: Thar, Roland, 52064 Aachen (DE); Fischer, Jan, 52064 Aachen (DE)
(74) Vertreter: Pellengahr, Maximilian Rudolf

(56) Entgegenhaltungen:
- WO-A1-02/061399
- WO-A1-2013/181679
- WO-A1-2015/143229
- JP-A- 2008 107 091
- US-A1- 2007 212 792
- US-A1- 2013 145 845

## Beschreibung

Die vorliegende Erfindung betrifft eine Vorrichtung zur Ermittlung mindestens eines Zustandsparameters, insbesondere der Konzentration und/oder des Partialdrucks, eines Analyten, insbesondere Sauerstoff, in einem gasförmigen Medium, insbesondere einem Luftvolumenstrom, umfassend
- mindestens eine Lichtquelle zur Emission von Licht,
- mindestens einen Lichtempfänger zur Erfassung von Licht,
- mindestens eine optische Sensoreinheit und
- mindestens ein Temperaturmessmittel zur zumindest mittelbaren Erfassung der Temperatur der Sensoreinheit,
wobei zumindest die Lichtquelle, der Lichtempfänger und das Temperaturmessmittel innerhalb eines Gehäuses und zumindest die Sensoreinheit außerhalb des Gehäuses angeordnet sind, wobei mindestens eine optische Eigenschaft der Sensoreinheit von dem Zustandsparameter des Analyten abhängt, wobei von der Sensoreinheit ausgehende Lichtstrahlen, die die Sensoreinheit infolge ihrer Bestrahlung mittels der Lichtquelle emittiert, mittels des Lichtempfängers erfassbar sind, wobei anhand auf diese Weise mittels des Lichtempfängers erfasster Daten ein Rückschluss auf den Zustandsparameter des Analyten in dem Medium gezogen werden kann.

Unter einer "Lichtquelle" wird im Sinne der vorliegenden Anmeldung eine Einrichtung verstanden, die dazu geeignet ist, Licht abzustrahlen. Hierbei muss es sich nicht notwendigerweise um für das menschliche Auge sichtbares Licht handeln. Stattdessen kann es sich beispielsweise ebenso um eine Infrarotstrahlung oder eine ultraviolette Strahlung handeln. Insbesondere ist als Lichtquelle eine LED denkbar.

Unter einem "Lichtempfänger" wird im Sinne der vorliegenden Anmeldung eine Einrichtung verstanden, die dazu geeignet ist, eine empfangene Lichtstrahlung in eine bestimmte Information umzusetzen. Insbesondere ist es denkbar, dass ein derartiger Lichtempfänger dazu geeignet ist, das empfangene Licht in eine elektrische Spannung oder einen elektrischen Strom umzusetzen. In jedem Fall muss es möglich sein, dass mittels des Lichtempfängers die Möglichkeit eröffnet wird, eine Information über das Licht zu erhalten, das den Lichtempfänger erreicht. Ein solcher Lichtempfänger kann insbesondere auch für Licht geeignet sein, dass das menschliche Auge nicht verarbeiten kann, beispielsweise eine Infrarotstrahlung. Beispielsweise kann ein Lichtempfänger von einer Photodiode gebildet sein.

Unter einer "optischen Sensoreinheit" wird im Sinne der vorliegenden Anmeldung eine Sensoreinheit verstanden, die durch Ausgabe optischer Informationen einen Rückschluss auf einen jeweils untersuchten Analyten zulässt. Im Sinne der vorliegenden Anmeldung sind hierunter insbesondere solche Sensoreinheiten zu verstehen, deren optischen Eigenschaften sich in Abhängigkeit des jeweiligen Zustandsparameters, beispielsweise der Konzentration, des jeweiligen Analyten verändern. Ein typisches Beispiel für eine solche Sensoreinheit ist eine solche, die lumineszierende Eigenschaften aufweist, wobei sich die Lumineszenz der Sensoreinheit in Abhängigkeit der Konzentration des jeweiligen Analyten verändert. Folglich kann durch Anregung einer solchen Sensoreinheit mittels der Lichtquelle und dem daraufhin von der Sensoreinheit abgestrahlten Licht ein Rückschluss darauf gezogen werden, welche Konzentration des Analyten in dem untersuchten gasförmigen Medium vorliegt. Alternativ zu lumineszierenden Sensoreinheiten sind auch absorbierende Sensoreinheiten sowie alle sonstigen technisch möglichen Sensoreinheiten denkbar. Eine absorbierende Sensoreinheit absorbiert in Abhängigkeit von dem Zustandsparameter unterschiedlich viel Licht, sodass durch die Untersuchung eines nicht-absorbierten Anteils ein Rückschluss auf den Zustandsparameter des Analyten gezogen werden kann.

Unter einem "Gehäuse" wird im Sinne der vorliegenden Anmeldung ein räumlich dezidierter Bereich verstanden, in dem zumindest in aller Regel sämtlichen elektronischen Bauteile der Vorrichtung untergebracht sind. Das Gehäuse kann geöffnet oder geschlossen ausgeführt sein, wobei letzteres insbesondere dann sinnvoll sein kann, wenn ein Feuchtigkeitseintrag in das Gehäuse und eine damit einhergehende Schädigung der elektronischen Bauteile der Vorrichtung befürchtet wird. Eine offene Ausgestaltung kann insbesondere den Vorteil eines Druckausgleichs zwischen den Bauteilen der Vorrichtung und der Umgebung bieten. Das Gehäuse kann insbesondere eine Trennwandung aufweisen, die eine unmittelbare räumliche Trennung zwischen einem Mediumraum und einem Innenraum des Gehäuses darstellt, wobei sich auf der Seite des Mediumraums das Medium und die Sensoreinheit befinden und auf der Seite des Innenraums die übrigen Komponenten der Vorrichtung angeordnet sind. Insbesondere die Trennwandung, möglicherweise das gesamte Gehäuse, kann von einem transparenten Material gebildet sein, sodass ein Durchtritt von Licht durch die Trennwandung ermöglicht ist.

### Stand der Technik

Im Stand der Technik sind Vorrichtungen der eingangs beschriebenen Art bereits bekannt. Sie werden beispielsweise dazu verwendet, die Konzentration von Sauerstoff in einem gasförmigen Volumenstrom zu bestimmen. Ein typischer Anwendungsfall hierfür kann beispielsweise die Bestimmungen der Sauerstoffkonzentration in der von einem Menschen ausgeatmeten Luft bestehen, um festzustellen, wie viel Sauerstoff der ausgeatmeten Luft zugesetzt werden muss, damit sie abermals für einen Menschen als frische Atemluft geeignet ist.

Die Sensoreinheiten derartiger Vorrichtungen bestehen typischerweise aus einer lumineszierenden chemischen Substanz, deren Lumineszenz sich in Abhängigkeit der Konzentration des Analyten ändert. Durch Anregung der Sensoreinheit mit Licht wird eine Lichtstrahlung ausgehend von der Sensoreinheit hervorgerufen, wobei Eigenschaften dieser von der Sensoreinheit ausgehenden Strahlung einen Rückschluss auf die Konzentration des Analyten in oder an der Sensoreinheit zulassen.

Im Stand der Technik sind verschiedene Vorrichtungen bekannt, die diese oder eine vergleichbare Technologie der Ermittlung der Konzentration eines Analyten in einem Medienstrom verwenden. Hierzu wird beispielhaft auf die Internationalen Patentanmeldungen WO 2005/100957 A1 und WO 2013/181679 A1 sowie auf die Deutsche Patentanmeldung DE 10 2014 112 972 A1 hingewiesen.

Ein typisches Problem bei der Ermittlung der Konzentration eines Analyten mittels einer optischen Sensoreinheit besteht darin, dass diese Art von Sensoreinheiten eine besonders hohe Sensibilität gegenüber sich ändernden Temperaturen aufweist. Diese Sensibilität äußert sich darin, dass sich die lumineszierenden Eigenschaften der Sensoreinheit infolge einer Veränderung der Temperatur gleichfalls verändern. Infolgedessen ist es verständlicherweise nicht möglich, aus einer Änderung der Lumineszenz der Sensoreinheit ohne Weiteres einen unmittelbaren Rückschluss auf die Ursache dieser Änderung zu ziehen, sodass insbesondere keine unmittelbare Aussage über die Konzentration des Analyten in dem zu untersuchenden Medium möglich ist.

Um diesem Problem zu begegnen, werden im Stand der Technik Temperatursensoren eingesetzt, die die Temperatur der Sensoreinheit fortwährend überwachen. Hierbei ist es von besonderer Bedeutung, dass eine Änderung der Temperatur der Sensoreinheit möglichst kurzfristig erfasst und somit im Rahmen der Auswertung der von der Sensoreinheit emittierten Strahlung berücksichtigt werden kann. Sollte nämlich ein bestimmter Zeitversatz zwischen der Änderung der Temperatur der Sensoreinheit und deren messtechnischer Erfassung mittels eines Temperaturmessmittel bestehen, muss zunächst bei Vorliegen einer Änderung der Temperatur der Sensoreinheit eine gewisse Übergangszeit gewährt werden, bevor eine verlässliche Aussage über die Konzentration des jeweils untersuchten Analyten möglich ist. Des Weiteren ist von Bedeutung, dass die Temperatur der Sensoreinheit möglichst wenig von der mittels des Temperaturmessmittels erfassten Temperatur abweicht. Abweichungen können insbesondere durch unterschiedliche Temperaturen des zu untersuchenden Mediums und des Gehäuses entstehen. Diese Abweichungen können einen negativen Einfluss auf die Genauigkeit der Messung des Analyten haben.

Um den beschriebenen Zeitversatz sowie etwaige Temperaturdifferenzen möglichst gering zu halten, wird im Stand der Technik beispielsweise vorgeschlagen, ein Temperaturmessmittel in unmittelbaren Kontakt mit der jeweiligen Sensoreinheit zu bringen und auf diese Weise eine Temperaturänderung der letzteren möglichst verzögerungsfrei und genau zu erfassen. Dieses Vorgehen birgt jedoch das Problem, dass das jeweilige Temperaturmessmittel aus dem Gehäuse der Elektronik der Vorrichtung herausgeführt werden muss und somit vergleichbar zu der Sensoreinheit in unmittelbaren Kontakt mit dem jeweils zu untersuchenden Medium tritt. Insbesondere besteht das Risiko, dass ein unbeabsichtigter Durchtritt des zu untersuchenden Mediums durch den aufgrund des Temperaturmessmittels geschaffenen Durchbruch des Gehäuses in das Gehäuse eintritt und die Elektronik schädigt.

Die Patentanmeldungen US 2007/212792 A1 und WO 2015/143229 A1 offenbaren die Merkmale des Oberbegriffes des beigefügten Anspruchs 1.

### Aufgabe

Der vorliegenden Erfindung liegt nunmehr die Aufgabe zugrunde, eine Vorrichtung bereitzustellen, die eine zuverlässige Ermittlung der Konzentration des jeweils untersuchten Analyten in dem jeweiligen Medium zulässt und gleichzeitig die konstruktiven Nachteile des Standes der Technik vermeidet.

### Lösung

Die Erfindung wird von der Vorrichtung des beigefügten Anspruchs 1 definiert,

Die zugrunde liegende Aufgabe wird ausgehend von der Vorrichtung der eingangs beschriebenen Art erfindungsgemäß durch eine thermische Isolierschicht gelöst, die dazu geeignet ist, die optische Sensoreinheit zumindest thermisch von dem zu untersuchenden Medium zu trennen. Diese Trennung soll zumindest derart erfolgen, dass Temperaturschwankungen des Medienvolumenstroms wenn überhaupt nur stark verzögert, vorzugsweise gar nicht, auf die Sensoreinheit übertragen werden. Vorteilhafterweise soll die Isolierschicht ferner etwaige sich ergebende Temperaturunterschiede zwischen dem Temperaturmessmittel im Inneren des Gehäuses und der Sensoreinheit außerhalb des Gehäuses minimieren, vorzugsweise vollständig vermeiden.

Unter einer "thermischen Isolierschicht" wird im Sinne der vorliegenden Anmeldung eine Schicht verstanden, deren primärer Zweck darin besteht, eine thermische Isolation der Sensoreinheit von dem zu untersuchenden Medium zu schaffen. Insoweit ist eine thermische Isolierschicht durch deren Eignung definiert, eine solche thermische Isolation bereitstellen zu können. Im Unterschied dazu sind Isolierschichten gemäß dem Stand der Technik, beispielsweise solche, die eine optische Isolierung der jeweiligen Sensoreinheit gewährleisten sollen, nicht als "thermische Isolierschicht" im Sinne der vorliegenden Anmeldung auffassbar, da sich diese bekannten Isolierschichten nicht als ebensolche eignen, da sie einen Durchgang von thermischer Energie von einer Seite der jeweiligen Schicht zu der jeweils anderen Seite kaum oder gar nicht verhindern können.

Es versteht sich zudem, dass jede Schicht eine gewisse Wärmekapazität aufweist, die die Schicht dazu befähigt, eine gewisse Menge thermischer Energie selbst aufzunehmen. Vorteilhafterweise ist die erfindungsgemäße thermische Isolierschicht derart ausgebildet, dass sie eine Wärmekapazität von mindestens 0,3 J/gK, vorzugsweise mindestens 0,5 J/gK, weiter vorzugsweise 0,8 J/gK, aufweist. Eine solche Isolierschicht ist besonders gut geeignet, eine Temperatur des zu untersuchenden Mediums an die Sensoreinheit weiterzuleiten, da die Isolierschicht selbst sich lediglich vergleichsweise träge in ihrer Temperatur verändern lässt.

Es versteht sich, dass die thermische Isolierschicht auf solche Weise mit der übrigen Vorrichtung zusammenwirken muss, dass eine unmittelbare Vorbeiführung des zu untersuchenden Medienvolumenstroms an der optischen Sensoreinheit unterbunden ist, sodass zumindest kein wesentlicher unmittelbarer Austausch von thermischer Energie, beispielsweise infolge von Konvektion, zwischen dem Medium und der Sensoreinheit möglich ist. Wäre letzterer trotz der thermischen Isolierschicht weiter vorhanden, wäre die Isolierschicht faktisch wirkungslos. Folglich versteht es sich, dass die Isolierschicht zumindest in irgendeiner Weise für den jeweils zu untersuchenden Analyten durchlässig sein muss, sodass trotz der Abschirmung des Medienvolumenstroms von der optischen Sensoreinheit zumindest die Migration einzelner Teilchen des Analyten ausgehend von dem Medium zu der optischen Sensoreinheit ermöglicht ist. Hierfür ist es gemäß der Erfindung vorgesehen, dass die thermische Isolierschicht offenporing porös ausgebildet ist. Grundsätzlich ist gleichwohl außerhalb des Schutzumfangs der beigefügten Ansprüche auch eine Diffusion eines Analyten durch einen Feststoff hindurch möglich. Die erfindungsgemäße poröse Ausgestaltung der Isolierschicht ist zur Erzeugung eines möglichst geringen Wärmedurchgangskoeffizienten der Isolierschicht vorteilhaft.

Die erfindungsgemäße Vorrichtung hat viele Vorteile. Insbesondere ist durch die Verwendung der thermischen Isolierschicht sichergestellt, dass eine Veränderung der Temperatur des jeweils untersuchten Mediums wenn überhaupt nur stark verzögert an die Sensoreinheit weitergegeben wird. Eine plötzliche Änderung der Temperatur der Sensoreinheit, wie sie im Stand der Technik regelmäßig befürchtet werden muss, kann somit nicht auftreten. Ferner sind kurzzeitige Schwankungen der Temperatur des untersuchten Mediums fast oder idealer Weise sogar vollständig ohne Einfluss auf die Temperatur der Sensoreinheit. Sollte es zu einer langfristigen Änderung der Temperatur des Mediums kommen, versteht es sich, dass sich dauerhaft auch die Temperatur der Sensoreinheit entsprechend anpassen wird. Aufgrund der thermischen Isolierschicht findet diese Änderung jedoch lediglich langsam und folglich kontrolliert statt. Die Erfassung einer solchen langsamen Änderung der Temperatur der Sensoreinheit ist mittels vergleichsweise simpler Temperaturmessmittel bereits zuverlässig ermittelbar, sodass fortwährend eine zuverlässige Ermittlung der Konzentration des Analyten in dem Medium möglich ist.

Ein weiterer Vorteil der erfindungsgemäßen Vorrichtung besteht darin, dass das mindestens eine Temperaturmessmittel, das zur Ermittlung der Temperatur der optischen Sensoreinheit verwendet wird, besonders einfach und folglich kostengünstig ausgeführt werden kann. Insbesondere ist kein aufwendiges Temperaturmessmittel erforderlich, dass zur Erfassung kurzfristiger Temperaturschwankungen der Sensoreinheit geeignet ist. Des Weiteren ist es bei der erfindungsgemäßen Vorrichtung vorteilhaft, dass das Temperaturmessmittel innerhalb des Gehäuses der Vorrichtung verbleiben kann, das heißt mindestens in einem gewissen Abstand zu der Sensoreinheit angeordnet ist, da sich letztere außerhalb des Gehäuses befindet. Trotz dieser "entfernten Anordnung" des Temperaturmessmittels relativ zu der Sensoreinheit kann aufgrund der lediglich langsamen Schwankungen der Temperatur der Sensoreinheit in jedem Fall eine zuverlässige Aussage über ebenjene Temperatur getroffen werden.

In einer vorteilhaften Ausgestaltung der erfindungsgemäßen Vorrichtung beträgt eine Dicke der thermischen Isolierschicht mindestens 0,5 mm, vorzugsweise mindestens 1,0 mm, weiter vorzugsweise mindestens 1,5 mm. Eine derartig ausgebildete Isolierschicht ist besonders gut geeignet, eine ausreichende thermische Isolation zwischen dem Medium und der Sensoreinheit zu gewährleisten.

Weiterhin kann eine solche Vorrichtung von besonderem Vorteil sein, bei dem das Material der Isolierschicht eine Wärmeleitfähigkeit von maximal 0,7 W/mK, vorzugsweise maximal 0,5 W/mK, weiter vorzugsweise maximal 0,3 W/mK, aufweist. Es versteht sich, dass die Verwendung eines solchen Materials besonders gut geeignet ist, eine thermische Isolationsfunktion wahrzunehmen.

Die erfindungsgemäße Vorrichtung weiter ausgestaltend beträgt der Wärmedurchgangskoeffizient der Isolierschicht maximal 400 W/m²K, vorzugsweise maximal 200 W/m²K, weiter vorzugsweise maximal 100 W/m²K. Hierbei ist es grundsätzlich unerheblich, ob der so beschriebene Wärmedurchgangskoeffizient aufgrund einer bestimmten Dicke der Isolierschicht oder aufgrund einer besonders geringen Wärmeleitfähigkeit des verwendeten Materials zustande kommt. Idealerweise weist die Isolierschicht sowohl eine bestimmte Dicke, eine vergleichsweise niedrige Wärmeleitfähigkeit als auch eine vergleichsweise hohe Wärmekapazität auf.

Vorteilhafterweise ist die Isolierschicht von einem hydrophoben Material gebildet, vorzugsweise von Polytetrafluorethylen (PTFE), insbesondere von gesintertem Polytetrafluorethylen, oder von Polyethylen (PE), insbesondere von gesintertem Polyethylen. Die Verwendung eines hydrophoben Materials ist insofern vorteilhaft, als ein Niederschlag von Flüssigkeit, insbesondere von Wasser, aus dem gasförmigen Medium auf einer Oberfläche der Isolierschicht kaum auftritt. Eine solche Flüssigkeitsschicht, die sich unbeabsichtigter Weise auf der Oberfläche der Isolierschicht niederschlägt, liefe insbesondere Gefahr, die Durchlässigkeit der Isolierschicht für den jeweils zu untersuchenden Analyten ausgehend von dem Medium zu der optischen Sensoreinheit zu blockieren und auf diese Weise die Vorrichtung insgesamt zu behindern. Die Ausführung der Isolierschicht aus hydrophobem Material vermeidet derartige Effekte. Polyethylen hat beispielsweise den weiteren Vorteil, dass es eine vergleichsweise große Wärmekapazität aufweist, die im Bereich von ca. 2,1 J/gK liegt.

Vorteilhafterweise weist die Isolierschicht eine Vielzahl kleinster Poren auf, deren maximale Porengröße 200 µm, vorzugsweise maximal 100 µm, weiter vorzugsweise maximal 50 µm, beträgt. Die Verwirklichung dieser Porengröße hat den besonderen Vorteil, dass eine "Strömung" des zu untersuchenden Mediums durch die thermische Isolierschicht hindurch quasi nicht möglich ist. Folglich ist insbesondere ein konvektiver Wärmeaustausch zwischen dem Medium und der optischen Sensoreinheit gleichfalls nicht möglich. Die Porosität ist gleichwohl ausreichend, um die Diffusion einzelner Teilchen des zu untersuchenden Analyten durch die thermische Isolierschicht hindurch zu erlauben und folglich die Konzentration des Analyten in oder an der Sensoreinheit innerhalb kürzester Zeit in Übereinstimmung mit der Konzentration des Analyten unmittelbar in dem Medienvolumenstrom zu erreichen. Sofern beispielsweise die Konzentration von Sauerstoff in einem Luftvolumenstrom untersucht werden soll, findet bei Verwendung der vorstehend beschriebenen Porengrößen eine Angleichung der Konzentration des Sauerstoffs beidseits der thermischen Isolierschicht innerhalb weniger Sekunden, insbesondere innerhalb von weniger als fünf Sekunden, statt.

In einer weiteren vorteilhaften Ausgestaltung der erfindungsgemäßen Vorrichtung ist die Isolierschicht derart relativ zu der Sensoreinheit positioniert, dass sie in unmittelbarem Kontakt mit letzterer steht. Das heißt, dass vorteilhafterweise zwischen einer der Sensoreinheit zugewandten Unterseite der Isolierschicht und einer korrespondierenden Oberfläche der Sensoreinheit keine planmäßige Freischicht vorliegt. Vorteilhafterweise bedeckt die Isolierschicht die gesamte Oberfläche der Sensoreinheit. Die Ausführung der Sensoreinheit und der Isolierschicht in unmittelbarem Kontakt miteinander hat den Vorteil, dass keine Gasschicht existiert, die einen Austausch des Analyten mit der Sensoreinheit verzögert, den Sensor also langsamer auf Änderungen der jeweiligen Zustandsvariable des Analyten werden lässt.

Unabhängig von der übrigen Gestaltung der Vorrichtung kann selbige dann besonders vorteilhaft sein, wenn die optische Sensoreinheit lumineszierende Eigenschaften aufweist, die von dem Zustandsparameter des Analyten in dem Medium abhängen. Eine solche Sensoreinheit kann infolge Anregung mittels Licht in einen lumineszierenden Zustand überführt werden, wobei die lumineszierenden Eigenschaften der Sensoreinheit mit der Konzentration des Analyten an oder in der Sensoreinheit korrelieren. Von der Sensoreinheit abgestrahltes Licht ist sodann mittels des Lichtempfängers erfassbar, wobei sich aus den Eigenschaften des abgestrahlten Lichts ein Rückschluss auf den Zustandsparameter des Analyten in dem Medium ziehen lässt. Alternativ oder zusätzlich zu einer lumineszierenden Sensoreinheit ist es ebenfalls denkbar, eine absorptive Sensoreinheit zu verwenden, die in Abhängigkeit des jeweiligen Zustandsparameters des Analyten unterschiedlich viel Licht absorbiert, sodass durch Beobachtung des von der Sensoreinheit infolge Bestrahlung reflektierten Lichts gleichfalls ein Rückschluss auf den jeweiligen Zustandsparameter des Analyten gezogen werden kann. Es versteht sich, dass zusätzlich oder alternativ sämtliche weiteren optischen Sensoreinheiten denkbar sind.

In einer vorteilhaften Ausführungsform der erfindungsgemäßen Vorrichtung umfasst selbige einen Messkanal, der sich ausgehend von einer Wandung des Gehäuses erstreckt und innerhalb dessen die Sensoreinheit und die Isolierschicht angeordnet sind. Die Isolierschicht ist dabei derart dichtend an eine innere Mantelfläche des Messkanals angeschlossen, dass ein Durchtritt zumindest des Analyten zwischen einer der Sensoreinheit abgewandten Oberseite und einer der Sensoreinheit zugewandten Unterseite der Isolierschicht ausschließlich durch die Isolierschicht hindurch möglich ist. Ein beschriebener Messkanal kann beispielsweise einen zylindrischen oder rechteckigen Querschnitt aufweisen, wobei die Isolierschicht bzw. ein die Isolierschicht bildendes Isolierelement vorzugsweise umlaufend dicht an die innere Mantelfläche des Messkanals angeschlossen ist. Es versteht sich, dass ausgehend von dem Gehäuse der Vorrichtung in Richtung des zu untersuchenden Mediums betrachtet sich die Isolierschicht an die Sensoreinheit anschließt, sodass sie letztere von dem Medium thermisch isolieren kann.

Der beschriebene Messkanal bietet vor allem den Vorteil, dass eine Anordnung der Isolierschicht in tatsächlich thermisch isolierender Weise relativ zu der Sensoreinheit besonders einfach möglich ist. Insbesondere kann die Sensoreinheit mittels gemeinsamer Wirkung einer umlaufenden Wandung des Messkanals und der Isolierschicht bzw. einem entsprechenden Isolierelement besonders gut strömungstechnisch von dem zu untersuchenden Medium abgeschirmt werden, sodass ein Austausch thermischer Energie zwischen dem Medium und der Sensoreinheit möglichst unterbunden ist.

Vorteilhafterweise ist bei Verwendung des beschriebenen Messkanals die Isolierschicht bzw. ein die Isolierschicht bildendes Isolierelement umlaufend mit der inneren Mantelfläche des Messkanals verklebt. Besonders vorteilhaft ist es dabei, wenn der Kleber hydrophobe Eigenschaften aufweist, sodass ein Niederschlag von kondensierter Flüssigkeit aus dem im Übrigen gasförmigen Medienvolumenstrom unterbunden ist.

Wie eingangs bereits erläutert, führt die thermische Isolierung der optischen Sensoreinheit von dem zu untersuchenden Medium dazu, dass die Temperatur der Sensoreinheit wesentlich geringeren Schwankungen unterlegen ist als bei im Stand der Technik bekannten Sensoreinheiten. Folglich bietet die erfindungsgemäße Vorrichtung den Vorteil, dass das jeweilig verwendete Temperaturmessmittel nicht in derselben Weise in unmittelbarem Kontakt mit der Sensoreinheit stehen muss, um in ausreichend kurzer Zeit eine Veränderung der Temperatur der Sensoreinheit erfassen zu können. Mithin bietet die erfindungsgemäße Vorrichtung den Vorteil, dass vergleichsweise einfache Temperaturmessmittel verwendet werden können, die zudem in einem gewissen Abstand von der Sensoreinheit, insbesondere innerhalb des Gehäuses der Vorrichtung, angeordnet werden können. Um eine Änderung der Temperatur der Sensoreinheit gleichwohl möglichst zeitversatzfrei zu erfassen, versteht es sich, dass es von besonderem Vorteil ist, wenn eine Wärmeübertragung von der Sensoreinheit zu dem Temperaturmessmittel möglichst widerstandsfrei stattfinden kann.

Bei Zugrundelegen einer Konstruktion der erfindungsgemäßen Vorrichtung, bei dem das Temperaturmessmittel innerhalb des Gehäuses und gleichzeitig die Sensoreinheit außerhalb des Gehäuses angeordnet sind, ist es daher von besonderem Vorteil, wenn zumindest die Sensoreinheit unmittelbar auf einer äußeren Oberfläche einer Wandung des Gehäuses angeordnet ist. Diese Ausgestaltung der Vorrichtung hat nämlich den Vorteil, dass infolge des unmittelbaren Kontakts zwischen dem Gehäuse und der Sensoreinheit gleichfalls eine unmittelbare Übertragung zwischen der Sensoreinheit und dem Gehäuse möglich ist. Insoweit ist mittels dieser erfindungsgemäße Anordnung sichergestellt, dass sich die Temperatur des Gehäuses, wie sie auf einer Innenseite der Wandung desselben gemessen werden kann, sehr zügig an die Temperatur der optischen Sensoreinheit angleicht. Folglich kann bei der erfindungsgemäßen Anordnung durch die Messung der Temperatur des Gehäuses auf der Innenseite von dessen Wandung besonders gut ein unmittelbarer Rückschluss auf die Temperatur der Sensoreinheit gezogen werden.

Dabei kann es weiterhin von besonderem Vorteil sein, wenn das Gehäuse insgesamt, zumindest jedoch eine die Sensoreinheit beherbergende Trennwandung desselben, einen Wärmedurchgangskoeffizienten von mindestens 50 W/m²K, vorzugsweise mindestens 75 W/m²K, weiter vorzugsweise mindestens 100 W/m²K, aufweisen.

Weiterhin ist es gemäß der Erfindung vorgesehen, dass ein Verhältnis zwischen dem Wärmedurchgangskoeffizienten der Isolierschicht und dem Wärmedurchgangskoeffizienten einer Trennwandung des Gehäuses maximal 1:1, vorzugsweise maximal 0,5:1, weiter vorzugsweise maximal 0,3:1, beträgt, wobei die Trennwandung einen Innenraum des Gehäuses von einem Mediumraum, in dem sich das Medium befindet, zumindest baulich trennt. Auf diese Weise ist sichergestellt, dass der Wärmeaustausch zwischen dem Temperaturmessmittel und der Sensoreinheit "schneller" vonstattengeht als zwischen dem Medium und der Sensoreinheit. Mit anderen Worten ist die Einflussnahme der Temperatur des Mediums auf die Sensoreinheit kleiner als die Möglichkeit zur Weiterleitung dieser Temperatur ausgehend von der Sensoreinheit zu dem Temperaturmessmittel.

Den vorstehenden Gedanken fortführend versteht es sich, dass eine Ausführung der erfindungsgemäßen Vorrichtung besonders vorteilhaft ist, bei der das im Innenraum des Gehäuses angeordneten Temperaturmessmittel sich in unmittelbarem Kontakt mit der inneren Oberfläche der Wandung des Gehäuses befindet. Vorzugsweise ist das Temperaturmessmittel dabei so platziert, dass ein minimaler Abstand zwischen einer dem Gehäuse zugewandten Oberfläche der Sensoreinheit und einer dem Gehäuse zugewandten Oberfläche des Temperaturmessmittels lediglich einer Dicke der Wandung des Gehäuses entspricht. Mit anderen Worten sind die Sensoreinheit und das Temperaturmessmittel vorteilhafterweise auf unmittelbar gegenüberliegenden Stellen der Wandung des Gehäuses angeordnet. Bei dieser Ausführung ist der "Weg", den die thermische Energie zwischen dem Temperaturmessmittel und der Sensoreinheit zurücklegen muss, ebenso minimal wie die Reaktionszeit des Temperaturmessmittels auf Änderungen der Temperatur der Sensoreinheit.

Als Temperaturmessmittel kann in einer vorteilhaften Ausgestaltung der Vorrichtung ein integrierter Temperatursensor verwendet werden, der vorzugsweise an einer Leiterplatte angeordnet ist. Derartige Temperatursensoren sind besonders zuverlässig und gleichzeitig günstig. Alternativ oder zusätzlich kann auch ein Temperaturmessmittel verwendet werden, welches mindestens einen Infrarotsensor aufweist. Ein solches Temperaturmessmittel ist insbesondere dazu geeignet, die Temperatur der Sensoreinheit oder der Trennwandung, die mit der Sensoreinheit zusammenwirkt, kontaktlos zu erfassen.

### Ausführungsbeispiele

Die erfindungsgemäße Vorrichtung ist nachstehend anhand eines Ausführungsbeispiels, das in den Figuren dargestellt ist, näher erläutert. Es zeigt:
- Fig. 1:: Einen Querschnitt durch eine erfindungsgemäße Vorrichtung und
- Fig. 2:: Einen Querschnitt durch einen Medienleitung, die mit der erfindungsgemäßen Vorrichtung gemäß Figur 1 versehen ist.

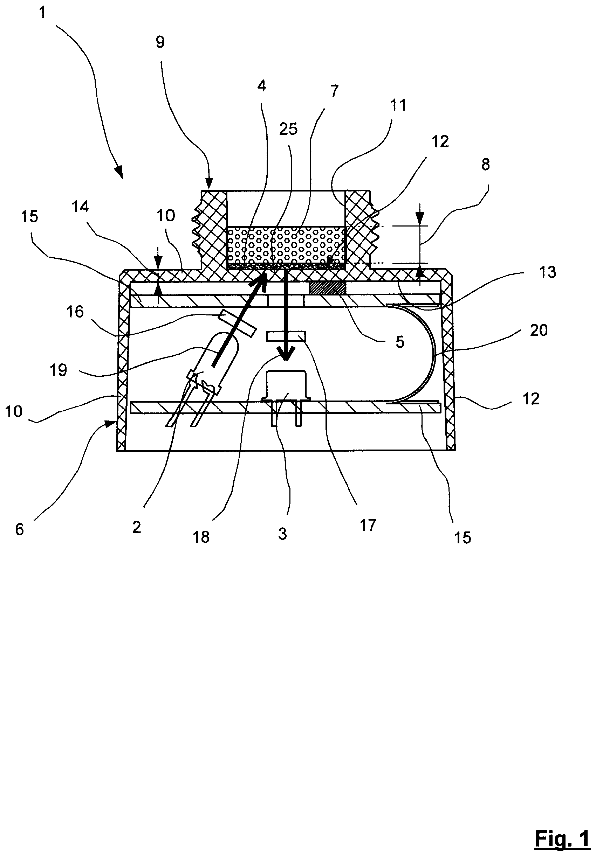

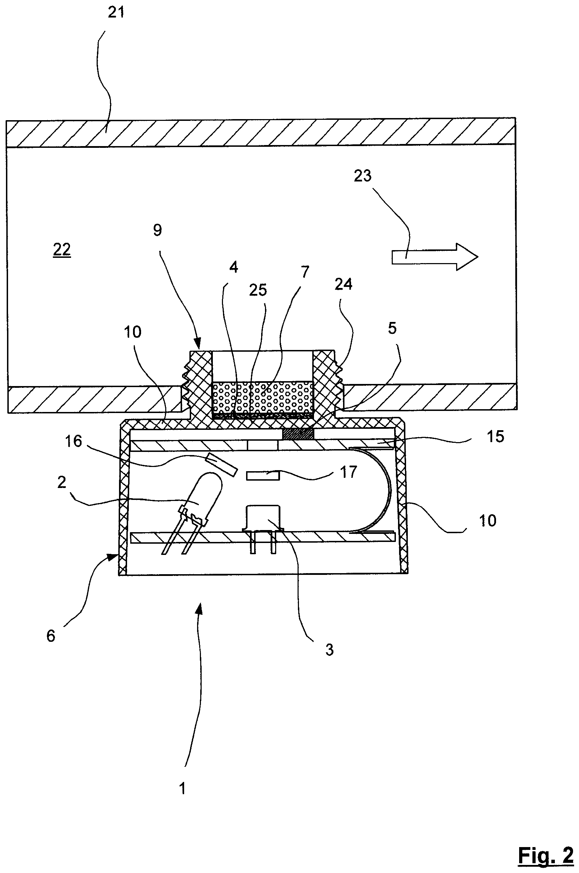

Das Ausführungsbeispiel, das in den **Figuren 1** **und** **2** dargestellt ist, umfasst eine erfindungsgemäße Vorrichtung **1,** die eine Lichtquelle **2,** einen Lichtempfänger **3** sowie eine Sensoreinheit **4** umfasst. Ferner weist die Vorrichtung **1** ein Temperaturmessmittel **5** auf, mittels dessen die Temperatur der Sensoreinheit **4** erfassbar ist. Die Vorrichtung **1** ist derart konstruiert, dass sämtliche elektronischen Bauteile innerhalb eines Gehäuses **6** angeordnet sind. Dieses Gehäuse **6** ist dadurch gekennzeichnet, dass es strömungstechnisch von dem jeweils zu untersuchenden Medium abgeschlossen ist, das heißt ein Innenraum des Gehäuses **6** mittels einer Wandung **10** von dem Medium abgeschirmt ist. Insbesondere ist der Innenraum mittels einer Trennwandung **25** unmittelbar von einer Mediumseite des Gehäuses **6** abgetrennt, wobei die Sensoreinheit **4** auf der Mediumseite der Trennwandung **25** angeordnet ist. Die Wandungen **10** des Gehäuses **6,** insbesondere die Trennwandung **25,** sind hier transparent ausgebildet. Die Lichtquelle **2,** der Lichtempfänger **3** als auch das Temperaturmessmittel **5** sind innerhalb des Gehäuses **6** angeordnet.

Die optische Sensoreinheit **4** ist außerhalb des Gehäuses **6** angeordnet, damit ein unmittelbarer Austausch zumindest einzelner Teilchen des Analyten zwischen dem Medium und der Sensoreinheit **4** möglich ist. In dem gezeigten Beispiel ist die Sensoreinheit **4** unmittelbar auf eine äußere Oberfläche **12** der Trennwandung **25** des Gehäuses **6** aufgebracht. Sie ist hier von einer lumineszierenden Farbschicht gebildet, die auf einem zugehörigen Träger angeordnet ist. Das Temperaturmessmittel **5** ist unmittelbar auf einer gegenüberliegenden inneren Oberfläche **13** der Trennwandung **25** angeordnet, sodass ein Abstand **14** zwischen der Sensoreinheit **4** und dem Temperaturmessmittel **5** minimal ist. Dies hat den Vorteil, dass eine Änderung der Temperatur der Sensoreinheit **4** möglichst verzögerungsfrei an das Temperaturmessmittel **5** weitergeleitet werden kann, sodass die mittels des Temperaturmessmittels **5** gemessene Temperatur zumindest im Wesentlichen mit der tatsächlichen Temperatur der Sensoreinheit **4** übereinstimmt. Die Trennwandung **25** weist einen Wärmedurchgangskoeffizienten von ca. 100 W/m²K auf.

Das Temperaturmessmittel **5** ist hier von einem integrierten Temperatursensor gebildet, der auf einer Leiterplatte **15** angeordnet ist. Diese Leiterplatte **15** ist in unmittelbarer Nähe zu der oberen Trennwandung **25** des Gehäuses **6** angeordnet. Sie ist ferner mittels einer Verbindungsleitung **20** mit einer weiteren Leiterplatte **15** verbunden, die sowohl die Lichtquelle **2** als auch den Lichtempfänger **3** hält. Mittels der Verbindungsleitung **20** ist eine elektrische Verbindung zwischen den Leiterplatten **15** geschaffen.

Die Lichtquelle **2** ist in dem gezeigten Beispiel von einer roten LED gebildet. Mittels der Lichtquelle **2** wird Licht in Richtung der optischen Sensoreinheit **4** emittiert. Dieses Licht ist in **Figur 1** mittels des dort dargestellten Pfeils **19** veranschaulicht. Um zu der optischen Sensoreinheit **4** zu gelangen, muss das ausgehend von der Lichtquelle **2** abgestrahlten Licht einen Anregungsfilter **16** passieren, der dazu geeignet ist, lediglich diejenigen Wellenlängen des abgestrahlten Lichts durchzulassen, die für die Anregung der Sensoreinheit **4** erforderlich sind. Damit das Licht auf die Sensoreinheit **4** treffen kann, versteht es sich, dass das Gehäuse **6** zumindest in dem mit der Sensoreinheit **4** zusammenwirkenden Bereich derart transparent ausgestaltet sein muss, dass das von der Lichtquelle **2** emittierte Licht auf die Sensoreinheit **4** treffen kann. Insbesondere kann das Gehäuse **6** insgesamt von einem transparenten Kunststoff gebildet sein.

Infolge der Anregung der Sensoreinheit **4** mittels des von der Lichtquelle **2** abgestrahlten Lichts emittiert die Sensoreinheit **4** ihrerseits Licht, wobei Eigenschaften des so von der Sensoreinheit **4** abgestrahlten Lichts unmittelbar mit den lumineszierenden Eigenschaften der Sensoreinheit **4** zusammenhängen, wobei die Lumineszenz wiederum von der Konzentration des Analyten an oder in der Sensoreinheit **4** abhängt. Das von der Sensoreinheit **4** abgestrahlte Licht wird sodann mittels des Lichtempfängers **3** empfangen. Das Licht ist in **Figur 1** symbolisch mittels des dort dargestellten Pfeils **18** veranschaulicht. Dem Lichtempfänger **3** ist noch ein Austrittsfilter **17** vorgeschaltet, der lediglich solche Wellenlängen des ausgehend von der Sensoreinheit **4** abgestrahlten Lichts passieren lässt, die für die Untersuchung der Konzentration des Analyten erforderlich sind.

Die lumineszierenden Eigenschaften der Sensoreinheit **4** hängen neben der Konzentration des jeweiligen Analyten ferner sehr stark von der Temperatur der Sensoreinheit **4** ab. Aus diesem Grund ist es überhaupt nur erforderlich, die Temperatur der Sensoreinheit **4** fortwährend mittels des Temperaturmessmittels **5** zu überwachen. Erfindungsgemäß ist auf einer dem Gehäuse **6** abgewandten Seite der Sensoreinheit **4** eine thermische Isolierschicht **7** angeordnet. Diese hat den primären technischen Effekt, dass die Sensoreinheit **4** thermisch von dem zu untersuchenden Medium isoliert wird, sodass sich eine Änderung der Temperatur des Mediums zumindest nicht unmittelbar, vorzugsweise gar nicht, auf die Temperatur der Sensoreinheit **4** auswirkt. Hierdurch wird der wesentliche Vorteil erreicht, dass die Änderung der Temperatur der Sensoreinheit **4** über die Zeit hinweg auf ein Minimum begrenzt werden kann und somit Temperaturschwankungen der Sensoreinheit **4** als Ursache für mögliche Messfehler wegfallen. Ferner bietet sich der besondere Vorteil, dass das verwendete Temperaturmessmittel **5** vergleichsweise einfach ausgeführt werden kann, da insbesondere plötzliche Änderungen der Temperatur der Sensoreinheit **4** um erhebliche Beträge nicht zu befürchten sind. Insbesondere ist es im Vergleich zum Stand der Technik nicht notwendig, das Temperaturmessmittel **5** in unmittelbaren Kontakt mit der Sensoreinheit **4** zu bringen und folglich aus dem Gehäuse **6** der Vorrichtung **1** herauszuführen. Stattdessen kann das Temperaturmessmittel **5** innerhalb des Gehäuses **6** verbleiben.

Die thermische Isolierschicht **7** weist hier eine Dicke **8** von ca. 2 mm auf. Demgegenüber weist der Träger der optischen Sensoreinheit **4** eine Dicke von ca. 0,1 mm auf. Die lumineszierende Farbschicht der Sensoreinheit **4** als solche weist lediglich eine Dicke von ca. 0,01 mm auf. Der Wärmedurchgangskoeffizient der Isolierschicht **7** beträgt hier 30 W/m²K. Somit beträgt das Verhältnis der Wärmedurchgangskoeffizienten der Isolierschicht **7** und des Gehäuses **6** (bzw. der Trennwandung **25**) hier 0,3:1.

Die thermische Isolierschicht **7** ist in dem gezeigten Beispiel von gesintertem Polytetrafluorethylen gebildet. Entsprechend weist die Isolierschicht **7** eine Vielzahl kleinster Poren auf, die insbesondere die Diffusion des Analyten ausgehend von einer dem Gehäuse **6** abgewandten Oberseite der Isolierschicht **7** zu einer dem Gehäuse **6** zugewandten Unterseite der Isolierschicht **7** erlauben. Die Poren der Isolierschicht **7** weisen hier eine Größe von ca. 30 µm auf. Im Übrigen ist die Isolierschicht **7** derart an der Vorrichtung **1** angeordnet, dass ein unmittelbarer Kontakt zwischen dem zu untersuchenden Medium und der Sensoreinheit **4** unterbunden ist. Hierzu verfügt die Vorrichtung **1** über einen Messkanal **9,** der sich ausgehend von dem Gehäuse **6** erstreckt. Die Sensoreinheit **4** ist an einem unteren, dem Gehäuse **6** zugewandten Ende des Messkanals **9** angeordnet und die Isolierschicht **7** gewissermaßen "darüber" platziert. Die Sensoreinheit **7** ist so positioniert, dass sich deren Unterseite in unmittelbarem Kontakt mit einer dem Gehäuse **6** abgewandten Oberfläche der Sensoreinheit **4** befindet. Mit anderen Worten befindet sich zwischen den einander zugewandten Oberflächen der Isolierschicht **7** und der Sensoreinheit **4** keine Freischicht, beispielsweise eine Luftschicht.

Die Verwendung von Polytetrafluorethylen ist hier insoweit von besonderem Vorteil, als es hydrophobe Eigenschaften aufweist, die die Ausbildung eines Flüssigkeitsfilms auf der Oberseite der Isolierschicht **7** verhindert. Hierdurch ist sichergestellt, dass die Poren der Isolierschicht **7,** durch die hindurch der jeweilige Analyt zu der Sensoreinheit **4** migrieren muss, nicht mittels einer sich niedergeschlagenen Flüssigkeit versiegelt werden. Zusätzlich zu der Isolierschicht **7** ist ferner auch der Kleber, mittels dessen in dem gezeigten Beispiel selbige mit der inneren Mantelfläche **11** des Messkanals **9** verbunden ist, hydrophob.

Beispielhaft ist in **Figur 2** eine denkbare Einbausituation einer erfindungsgemäßen Vorrichtung **1** dargestellt. Hier umfasst der Messkanal **9** an einer äußeren Mantelfläche ein Außengewinde **24,** das mit einem komplementären Innengewinde einer Medienleitung **21** zusammenwirkt. In der Medienleitung **21** wird ein jeweils zu untersuchendes Medium **22,** beispielsweise Atemluft, in eine Strömungsrichtung **23** geführt. Das Medium **22** strömt dabei unmittelbar an der Vorrichtung **1** entlang, wobei ein unmittelbarer Austausch eines jeweiligen Analyten zwischen dem Medium **22** und der Sensoreinheit **4** durch die Isolierschicht **7** hindurch möglich ist. Beispielsweise ist es denkbar, dass die Konzentration von Sauerstoff in dem Medium **22** untersucht werden soll. Aus der Darstellung ergibt sich ohne Weiteres, dass das Medium **22** mittels der Isolierschicht **7** zumindest thermisch von der Sensoreinheit **4** entkoppelt ist, sodass etwaige Temperaturschwankungen des Mediums **22** keine unmittelbare Auswirkung auf die Temperatur der Sensoreinheit **4** haben. Gleichzeitig erlaubt die Porosität der Isolierschicht **7** eine besonders zügige Diffusion der einzelnen Teilchen des zu untersuchenden Analyten durch die Isolierschicht **7** hindurch, sodass sich die Konzentration des jeweiligen Analyten in bzw. an der Sensoreinheit **4** innerhalb kürzester Zeit, insbesondere innerhalb von weniger als zwei Sekunden, an die Konzentration des Analyten in dem Medium **22** angepasst hat. Insoweit ist die erfindungsgemäße Isolierschicht **7** für eine besonders kurze Ansprechzeit der Sensoreinheit **4** nicht hinderlich.

### Bezugszeichenliste

- 1: Vorrichtung
- 2: Lichtquelle
- 3: Lichtempfänger
- 4: Sensoreinheit
- 5: Temperaturmessmittel
- 6: Gehäuse
- 7: Isolierschicht
- 8: Dicke
- 9: Messkanal
- 10: Wandung
- 11: Innere Mantelfläche
- 12: Äußere Oberfläche
- 13: Innere Oberfläche
- 14: Abstand
- 15: Leiterplatte
- 16: Anregungsfilter
- 17: Austrittsfilter
- 18: Pfeil
- 19: Pfeil
- 20: Verbindungsleitung
- 21: Medienleitung
- 22: Medium
- 23: Strömungsrichtung
- 24: Außengewinde
- 25: Trennwandung

## Patentansprüche

1. Vorrichtung (1) zur Ermittlung mindestens eines Zustandsparameters, insbesondere der Konzentration und/oder des Partialdrucks, eines Analyten in einem gasförmigen Medium, umfassend
- ein Gehäuse (6),
- mindestens eine Lichtquelle (2) zur Emission von Licht,
- mindestens einen Lichtempfänger (3) zur Erfassung von Licht,
- mindestens eine optische Sensoreinheit (4) und
- mindestens ein Temperaturmessmittel (5) zur zumindest mittelbaren Erfassung der Temperatur der Sensoreinheit (4),
wobei das Gehäuse (6) eine Trennwandung (25) aufweist, die einen Innenraum des Gehäuses (6) von einem Mediumraum, in dem sich das Medium befindet, zumindest baulich trennt,
wobei zumindest die Lichtquelle (2), der Lichtempfänger (3) und das Temperaturmessmittel (5) bezogen auf die Trennwandung (25) auf der Seite des Innenraums innerhalb des Gehäuses (6) und zumindest die Sensoreinheit (4) bezogen auf die Trennwandung (25) auf der Seite des Mediumraums außerhalb des Gehäuses (6) angeordnet sind,
wobei mindestens eine optische Eigenschaft der Sensoreinheit (4) von dem Zustandsparameter des Analyten abhängt,
wobei von der Sensoreinheit (4) ausgehende Lichtstrahlen, die die Sensoreinheit (4) infolge ihrer Bestrahlung mittels der Lichtquelle (2) emittiert, mittels des Lichtempfängers (3) erfassbar sind,
wobei anhand auf diese Weise mittels des Lichtempfängers (3) erfasster Daten ein Rückschluss auf den Zustandsparameter des Analyten in dem Medium gezogen werden kann,
**gekennzeichnet durch**
mindestens eine thermische Isolierschicht (7), die auf einer dem Gehäuse (6) abgewandten Seite der Sensoreinheit (4) angeordnet ist und dazu geeignet ist, die Sensoreinheit (4) zumindest thermisch von dem Medium zu isolieren,
wobei ein Verhältnis zwischen dem Wärmedurchgangskoeffizienten der thermischen Isolierschicht (7) und dem Wärmedurchgangskoeffizienten der Trennwandung (25) des Gehäuses (6) maximal 1:1 beträgt,
wobei die thermische Isolierschicht (7) offenporig porös ausgebildet ist

2. Vorrichtung (1) nach Anspruch 1, **dadurch gekennzeichnet, dass** eine Dicke (8) der thermischen Isolierschicht (7) mindestens 0,5 mm, vorzugsweise mindestens 1,0 mm weiter vorzugsweise mindestens 1,5 mm, beträgt.

3. Vorrichtung (1) nach Anspruch 1 oder 2, **dadurch gekennzeichnet, dass** ein Material, von dem die thermische Isolierschicht (7) gebildet ist, eine Wärmeleitfähigkeit von maximal 0,7 W/mK, vorzugsweise maximal 0,5 W/mK, weiter vorzugsweise maximal 0,3 W/mK, aufweist.

4. Vorrichtung (1) nach einem der Ansprüche 1 bis 3, **dadurch gekennzeichnet, dass** der Wärmedurchgangskoeffizient der thermischen Isolierschicht (7) maximal 400 W/m²K vorzugsweise maximal 200 W/m²K, weiter vorzugsweise maximal 100 W/m²K, beträgt.

5. Vorrichtung (1) nach einem der Ansprüche 1 bis 4, **dadurch gekennzeichnet, dass** die thermische Isolierschicht (7) von einem hydrophoben Material gebildet ist insbesondere von Polytetrafluorethylen, vorzugsweise von gesintertem Polytetrafluorethylen.

6. Vorrichtung (1) nach einem der Ansprüche 1 bis 5, **dadurch gekennzeichnet, dass** die thermische Isolierschicht (7) Poren mit einer maximalen Porengröße von 200 µm vorzugsweise maximal 100 µm, weiter vorzugsweise maximal 50 µm, aufweist.

7. Vorrichtung (1) nach einem der Ansprüche 1 bis 6, **dadurch gekennzeichnet, dass** die thermische Isolierschicht (7) in unmittelbarem Kontakt mit der Sensoreinheit (4) steht, wobei die thermische Isolierschicht (7) vorzugsweise eine gesamte, dem Gehäuse (6) abgewandte Oberfläche der Sensoreinheit (4) bedeckt.

8. Vorrichtung (1) nach einem der Ansprüche 1 bis 7, **dadurch gekennzeichnet, dass** die optische Sensoreinheit (4) lumineszierende Eigenschaften aufweist, die von dem Zustandsparameter des Analyten in dem Medium abhängen, wobei infolge Anregung mittels Licht in einen lumineszierenden Zustand überführbar ist, wobei die lumineszierenden Eigenschaften der Sensoreinheit (4) mit der Konzentration des Analyten an oder in der Sensoreinheit (4) korrelieren, wobei von der Sensoreinheit (4) infolge der Anregung emittiertes Licht mittels des Lichtempfängers (3) erfassbar ist, sodass anhand so erfasster Daten ein Rückschluss auf den Zustandsparameter des Analyten in dem Medium gezogen werden kann,

9. Vorrichtung (1) nach einem der Ansprüche 1 bis 8, **gekennzeichnet durch** einen Messkanal (9), der sich ausgehend von einer Wandung (10) des Gehäuses (6) erstreckt und innerhalb dessen die Sensoreinheit (4) und die thermische Isolierschicht (7) angeordnet sind, wobei die thermische Isolierschicht (7) derart dichtend an eine innere Mantelfläche (11) des Messkanals (9) angeschlossen ist, dass ein Durchtritt zumindest des Analyten zwischen einer der Sensoreinheit (4) abgewandten Seite und einer der Sensoreinheit (4) zugewandten Seite der thermischen Isolierschicht (7) ausschließlich durch die thermische Isolierschicht (7) hindurch möglich ist

10. Vorrichtung (1) nach Anspruch 9, **dadurch gekennzeichnet, dass** die thermische Isolierschicht (7) umlaufend mit der inneren Mantelfläche (11) des Messkanals (9) verklebt ist, wobei vorzugsweise der Kleber hydrophobe Eigenschaften aufweist.

11. Vorrichtung (1) nach einem der Ansprüche 1 bis 10, **dadurch gekennzeichnet, dass** die Sensoreinheit (4) unmittelbar auf einer äußeren Oberfläche (12) einer Wandung (10) des Gehäuses (6) angeordnet ist.

12. Vorrichtung (1) nach einem der Ansprüche 1 bis 11, **dadurch gekennzeichnet, dass** das mindestens eine Temperaturmessmittel (5) in unmittelbarem Kontakt mit einer inneren Oberfläche (13) einer Wandung (10) des Gehäuses (6) steht, wobei das Temperaturmessmittel (5) vorzugweise so platziert ist, dass ein minimaler Abstand (14) zwischen einer dem Gehäuse (6) zugwandten Oberfläche der Sensoreinheit (4) und dem Temperaturmessmittel (5) einer Dicke der Wandung (10) des Gehäuses (6) entspricht.

13. Vorrichtung (1) nach einem der Ansprüche 1 bis 12, **dadurch gekennzeichnet, dass** ein Verhältnis zwischen dem Wärmedurchgangskoeffizienten der thermischen Isolierschicht (7) und dem Wärmedurchgangskoeffizienten einer Trennwandung (25) des Gehäuses (6) maximal 0,5:1, vorzugsweise maximal 0,3:1, beträgt.

14. Vorrichtung (1) nach einem der Ansprüche 1 bis 13, **dadurch gekennzeichnet, dass** das Temperaturmessmittel (5) von einem integrierten Temperatursensor gebildet ist, der vorzugsweise an einer Leiterplatte (15) angeordnet ist.

15. Vorrichtung (1) nach einem der Ansprüche 1 bis 14, **dadurch gekennzeichnet, dass** die thermische Isolierschicht (7) eine Wärmekapazität von mindestens 0,3 J/gK vorzugsweise mindestens 0,5 J/gK, weiter vorzugsweise mindestens 0,8 J/gK, aufweist.

## Claims

1. Device (1) for determining at least one state parameter, in particular the concentration and/or the partial pressure, of an analyte in a gaseous medium, comprising
- a housing (6),
- at least one light source (2) for emitting light,
- at least one light receiver (3) for capturing light,
- at least one optical sensor unit (4) and
- at least one temperature measurement means (5) for capturing at least indirectly the temperature of the sensor unit (4),
wherein the housing (6) has a dividing wall (25) that separates at least structurally an interior space of the housing (6) from a medium space in which the medium is located,
wherein at least the light source (2), the light receiver (3) and the temperature measurement means (5) are arranged on the side of the interior space inside the housing (6) relative to the dividing wall (25) and at least the sensor unit (4) is arranged on the side of the medium space outside the housing (6) relative to the dividing wall (25),
wherein at least one optical property of the sensor unit (4) depends on the state parameter of the analyte, wherein light rays emanating from the sensor unit (4), which the sensor unit (4) emits as a result of its irradiation by means of the light source (2), can be captured by means of the light receiver (3),
wherein, based on data captured in this manner by means of the light receiver (3), it is possible to infer the state parameter of the analyte in the medium,
**characterized by**
at least one thermal insulation layer (7), which is arranged on a side of the sensor unit (4) that faces away from the housing (6) and which is suitable for insulating the sensor unit (4) at least thermally from the medium, wherein a ratio between the heat transfer coefficient of the thermal insulation layer (7) and the heat transfer coefficient of the dividing wall (25) of the housing (6) is at most 1:1, wherein the thermal insulation layer (7) is configured to be porous with open pores.

2. The device (1) according to claim 1, **characterized in that** a thickness (8) of the thermal insulation layer (7) is at least 0.5 mm, preferably at least 1.0 mm, even more preferably at least 1.5 mm.

3. The device (1) according to claim 1 or 2, **characterized in that** a material from which the thermal insulation layer (7) is formed has a thermal conductivity of at most 0.7 W/mK, preferably at most 0.5 W/mK, even more preferably at most 0.3 W/mK.

4. The device (1) according to one of claims 1 to 3, **characterized in that** the heat transfer coefficient of the thermal insulation layer (7) is at most 400 W/m²K, preferably at most 200 W/m²K, even more preferably at most 100 W/m²K.

5. The device (1) according to one of claims 1 to 4, **characterized in that** the thermal insulation layer (7) is formed from a hydrophobic material, in particular polytetrafluoroethylene, preferably sintered polytetrafluoroethylene.

6. The device (1) according to one of claims 1 to 5, **characterized in that** the thermal insulation layer (7) has pores with a pore size of at most 200 µm, preferably at most 100 µm, even more preferably at most 50 µm.

7. The device (1) according to one of claims 1 to 6, **characterized in that** the thermal insulation layer (7) is in direct contact with the sensor unit (4), wherein the thermal insulation layer (7) preferably covers an entire surface of the sensor unit (4) that faces away from the housing (6).

8. The device (1) according to one of claims 1 to 7, **characterized in that** the optical sensor unit (4) has luminescent properties that depend on the state parameter of the analyte in the medium, wherein it can be converted into a luminescent state as a result of excitation by means of light, wherein the luminescent properties of the sensor unit (4) correlate with the concentration of the analyte on or in the sensor unit (4), wherein light emitted by the sensor unit (4) as a result of the excitation can be detected by means of the light receiver (3) so that, based on data captured in this manner, it is possible to infer the state parameter of the analyte in the medium.

9. The device (1) according to one of claims 1 to 8, **characterized by** a measuring channel (9), which extends from a wall (10) of the housing (6) and inside which the sensor unit (4) and the thermal insulation layer (7) are arranged, wherein the thermal insulation layer (7) is connected to an inner jacket surface (11) of the measuring channel (9) so as to seal the latter so that a passage of at least the analyte between a side of the thermal insulation layer (7) that faces away from the sensor unit (4) and a side of the thermal insulation layer (7) that faces the sensor unit (4) is solely possible through the thermal insulation layer (7).

10. The device (1) according to claim 9, **characterized in that** the thermal insulation layer (7) is glued around its circumference to the inner jacket surface (11) of the measuring channel (9), wherein the glue preferably has hydrophobic properties.

11. The device (1) according to one of claims 1 to 10, **characterized in that** the sensor unit (4) is arranged directly on an outer surface (12) of a wall (10) of the housing (6).

12. The device (1) according to one of claims 1 to 11, **characterized in that** at least one temperature measurement means (5) is in direct contact with an inner surface (13) of a wall (10) of the housing (6), wherein the temperature measurement means (5) is preferably placed so that a minimum distance (14) between a surface of the sensor unit (4) that faces the housing (6) and the temperature measurement means (5) corresponds to a thickness of the wall (10) of the housing (6).

13. The device (1) according to one of claims 1 to 12, **characterized in that** a ratio between the heat transfer coefficient of the thermal insulation layer (7) and the heat transfer coefficient of a dividing wall (25) of the housing (6) is at most 0.5:1, preferably at most 0.3:1.

14. The device (1) according to one of claims 1 to 13, **characterized in that** the temperature measurement means (5) is formed by an integrated temperature sensor, which is preferably arranged on a printed circuit board (15).

15. The device (1) according to one of claims 1 to 14, **characterized in that** the thermal insulation layer (7) has a heat capacity of at least 0.3 J/gK, preferably at least 0.5 J/gK, even more preferably at least 0.8 J/gK.

## Revendications

1. Dispositif (1) permettant de déterminer au moins un paramètre d'état, en particulier la concentration et/ou la pression partielle d'un analyte dans un milieu gazeux, comprenant
- un boîtier (6),
- au moins une source de lumière (2) pour l'émission de lumière,
- au moins un récepteur de lumière (3) pour saisir la lumière,
- au moins une unité de détection optique (4) et
- au moins un moyen de mesure de température (5) pour la saisie au moins indirecte de la température de l'unité de détection (4),
sachant que le boîtier (6) comporte une paroi de séparation (25), qui sépare au moins du point de vue construction un espace intérieur du boîtier (6) d'un compartiment de milieu dans lequel se trouve le milieu,
sachant qu'au moins la source de lumière (2), le récepteur de lumière (3) et le moyen de mesure de température (5) par rapport à la paroi de séparation (25) sont disposés sur le côté du compartiment intérieur à l'intérieur du boîtier (6) et au moins l'unité de détection (4) par rapport à la paroi de séparation (25) est disposée sur le côté du compartiment de milieu en dehors du boîtier (6),
sachant qu'au moins une propriété optique de l'unité de détection (4) dépend du paramètre d'état de l'analyte,
sachant que les rayons lumineux émis par l'unité de détection (4), qu'émet l'unité de détection (4) en raison de son rayonnement au moyen de la source de lumière (2), peuvent être saisis au moyen du récepteur de lumière (3),
sachant qu'une déduction concernant le paramètre d'état de l'analyte dans le milieu peut être tirée à l'aide des données saisies de cette manière au moyen du récepteur de lumière (3),
**caractérisé par**
au moins une couche d'isolation thermique (7), qui est disposée sur un côté opposé au boîtier (6) de l'unité de détection (4) et qui est apte à isoler au moins thermiquement l'unité de détection (4) du milieu,
sachant qu'un rapport entre le coefficient de transmission thermique de la couche d'isolation thermique (7) et le coefficient de transmission thermique de la paroi de séparation (25) du boîtier (6) est au maximum de 1:1,
sachant que la couche d'isolation thermique (7) est constituée poreuse à pores ouverts.

2. Dispositif (1) selon la revendication 1, **caractérisé en ce qu'**une épaisseur (8) de la couche d'isolation thermique (7) est au moins de 0,5 mm, de préférence au moins de 1,0 mm, de préférence en plus au moins de 1,5 mm.

3. Dispositif (1) selon la revendication 1 ou 2, **caractérisé en ce qu'un** matériau, dans lequel est formée la couche d'isolation thermique (7), comporte une conductibilité thermique au maximum de 0,7 W/mK, de préférence au maximum de 0,5 W/mK, de préférence en plus au maximum de 0,3 W/mK.

4. Dispositif (1) selon l'une quelconque des revendications 1 à 3, **caractérisé en ce que** le coefficient de transmission thermique de la couche d'isolation thermique (7) est au maximum de 400 W/ m²K, de préférence au maximum de 200 W/m²K, de préférence en plus au maximum de 100 W/m²K.

5. Dispositif (1) selon l'une quelconque des revendications 1 à 4, **caractérisé en ce que** la couche d'isolation thermique (7) est formée d'un matériau hydrophobe, en particulier de polytétrafluoréthylène, de préférence de polytétrafluoréthylène fritté.

6. Dispositif (1) selon quelconque des revendications 1 à 5, **caractérisé en ce que** la couche d'isolation thermique (7) comporte des pores avec une taille de pore maximale de 200 µm, de préférence au maximum de 100 µm, de préférence en plus au maximum de 50 µm.

7. Dispositif (1) selon l'une quelconque des revendications 1 à 6, **caractérisé en ce que** la couche d'isolation thermique (7) est en contact direct avec l'unité de détection (4), sachant que la couche d'isolation thermique (7) couvre de préférence une surface totale de l'unité de détection (4), opposé au boîtier (6).

8. Dispositif (1) selon l'une quelconque des revendications 1 à 7, **caractérisé en ce que** l'unité de détection optique (4) comporte des propriétés luminescentes, qui dépendent du paramètre d'état de l'analyte dans le milieu, sachant qu'il est possible de passer dans un état luminescent par suite de l'excitation au moyen de lumière, sachant que les propriétés luminescentes de l'unité de détection (4) peuvent être mises en corrélation avec la concentration de l'analyte sur ou dans l'unité de détection (4), sachant que la lumière émise par l'unité de détection (4) en raison de l'excitation peut être saisie au moyen du récepteur de lumière (3) de telle sorte qu'une déduction concernant le paramètre d'état de l'analyte dans le milieu peut être tirée à l'aide des données ainsi saisies.

9. Dispositif (1) selon l'une quelconque des revendications 1 à 8, **caractérisé par** un canal de mesure (9), qui s'étend en partant d'une paroi (10) du boîtier (6) et à l'intérieur duquel l'unité de détection (4) et la couche d'isolation thermique (7) sont disposées, sachant que la couche d'isolation thermique (7) est raccordée hermétiquement à une surface d'enveloppe intérieure (11) du canal de mesure (9) de telle manière qu'un passage au moins de l'analyte entre un côté opposé à l'unité de détection (4) et un côté tourné vers l'unité de détection (4) de la couche d'isolation thermique (7) n'est exclusivement possible qu'à travers la couche d'isolation thermique (7).

10. Dispositif (1) selon la revendication 9, **caractérisé en ce que** la couche d'isolation thermique (7) est collée de façon périphérique avec la surface d'enveloppe intérieure (11) du canal de mesure (9), sachant de préférence que l'adhésif comporte des propriétés hydrophobes.

11. Dispositif (1) selon l'une quelconque des revendications 1 à 10, **caractérisé en ce que** l'unité de détection (4) est disposée directement sur une surface extérieure (12) d'une paroi (10) du boîtier (6).

12. Dispositif (1) selon l'une quelconque des revendications 1 à 11, **caractérisé en ce qu'**au moins un moyen de mesure de température (5) est en contact direct avec une surface intérieure (13) d'une paroi (10) du boîtier (6), sachant que le moyen de mesure de température (5) est de préférence placé de telle sorte qu'une distance (14) minimale entre une surface de l'unité de détection (4) tournée vers le boîtier (6) et le moyen de mesure de température (5) correspond à une épaisseur de la paroi (10) du boîtier (6).

13. Dispositif (1) selon l'une quelconque des revendications 1 à 12, **caractérisé en ce qu'**un rapport entre le coefficient de transmission thermique de la couche d'isolation thermique (7) et le coefficient de transmission thermique d'une paroi de séparation (25) du boîtier (6) est au maximum de 0,5:1, de préférence au maximum de 0,3:1.

14. Dispositif (1) selon l'une quelconque des revendications 1 à 13, **caractérisé en ce que** le moyen de mesure de température (5) est formé par un capteur de température intégré, qui est disposé de préférence sur une carte de circuits imprimés (15).

15. Dispositif (1) selon l'une quelconque des revendications 1 à 14 **caractérisé en ce que** la couche d'isolation thermique (7) comporte une capacité thermique d'au moins 0,3 J/gK, de préférence d'au moins 0,5 J/gK, de préférence en plus d'au moins 0,8 J/gK.
